# EUROPÄISCHE PATENTSCHRIFT

(11) **EP 3 727 538 B1**
(45) Veröffentlichungstag und Bekanntmachung des Hinweises auf die Patenterteilung: **31.08.2022**
(21) Anmeldenummer: 18826294.3
(22) Anmeldetag: 18.12.2018
(51) Int. Cl.: A61F 2/20, A61M 16/04, A61M 16/20

(54) **TRACHEAOSTOMIEKANÜLE MIT EINER PHONATIONSÖFFNUNG**
TRACHEOSTOMY CANNULA HAVING A PHONATION OPENING
CANULE DE TRACHÉOTOMIE COMPORTANT UNE OUVERTURE DE PHONATION

(30) Priorität: 22.12.2017 DE 102017131137
(43) Veröffentlichungstag der Anmeldung: 28.10.2020
(73) Patentinhaber: Tracoe medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2018/085438
(87) Internationale Veröffentlichungsnummer: WO 2019/121645

(56) Entgegenhaltungen:
- EP-A1- 2 322 240
- EP-A1- 2 801 384
- EP-A1- 2 810 618
- WO-A1-2016/038323
- WO-A2-99/32169
- DE-A1-102016 002 841
- GB-A- 2 340 401
- JP-A- 2016 026 542
- US-A- 4 280 492
- US-A- 4 459 984
- US-B1- 6 722 367

## Beschreibung

Die vorliegende Erfindung betrifft eine Tracheostomiekanüle mit einem Kanülenrohr, wobei das Kanülenrohr ein proximales Ende mit einer proximalen Öffnung, ein distales Ende mit einer distalen Öffnung, sowie eine Phonationsöffnung in der Wand des Kanülenrohrs zwischen dem distalen Ende und dem proximalen Ende aufweist.

Die Phonationsöffnung kann eine einzelne, größere Öffnung sein oder aus einer Mehrzahl kleinerer, siebartig angeordneter Öffnungen bestehen, deren Querschnitt für den jeweils beim Atmen auftretenden Luftstrom ausreichend ist. Wenn die durch die Phonationsöffnung strömende Atemluft beim Ausatmen am Kehlkopf und den Stimmbändern des Patienten vorbeigeleitet wird, ermöglicht die Phonationsöffnung das Sprechen.

Derartige Tracheostomiekanülen werden in die Trachea eines Patienten eingeführt, um den Patienten mit Luft bzw. Atemgas zu versorgen. Das proximale Ende der Tracheostomiekanüle ist außerhalb des Patienten mit einer Beatmungsvorrichtung verbindbar oder, vorzugsweise über einen Filter, mit der Umgebung verbunden, während das distale Ende der Tracheostomiekanüle in die Trachea einführbar ist. Das die zwei Öffnungen verbindende Kanülenrohr dient der Hindurchleitung des Atemgases (Atemluft). Das Kanülenrohr wird durch eine künstlich im Kehlkopfbereich eines Patienten erzeugte Öffnung (Tracheostoma) in die Trachea des Patienten eingeführt. Für die künstliche Beatmung eines Patienten wird meist eine Innenkanüle ohne Phonationsöffnung in die Tracheostomiekanüle eingesetzt. Eingesetzt werden kann jedoch auch eine Innenkanüle, die ein oder mehrere zu der Phonationsöffnung der Außenkanüle korrespondierende Phonationsöffnungen aufweist. Solche Innenkanülen werden typischerweise eingesetzt, um Patienten das Sprechen zu erleichtern.

Soll die Tracheostomiekanüle nach längerer Zeit wieder aus der Trachea eines Patienten entfernt werden und der Patient durch die natürlichen Atemwege atmen, ist es erforderlich, dass der Patient von der Tracheostomiekanüle entwöhnt und an einen höheren Atemwiderstand der natürlichen Atemwege gewöhnt wird. Häufig wird hierzu die proximale Öffnung der Tracheostomiekanüle mit einem Sprechventil oder einer Verschlusskappe verschlossen.

Auch wenn die Tracheostomiekanüle dauerhaft in dem Tracheostoma verbleiben muss, erleichtert ein Sprechventil an der Kanüle für den Patienten die Kommunikation mit der Umgebung. Ein Sprechventil ist ein Einwegventil am proximalen Ende der Kanüle, welches ein Einatmen durch die proximale Öffnung der Tracheostomiekanüle erlaubt, ein Ausatmen durch die proximale Öffnung der Tracheostomiekanüle jedoch verhindert. Bei einem verschlossenen proximalen Ende müsste, wenn keine Phonationsöffnung vorhanden wäre, die Atemluft beim Ausatmen durch den schmalen Spalt zwischen der Außenseite der Kanüle und der Trachealwand hindurch und weiter durch die natürlichen Atemwege strömen.

Damit der Atemwiderstand reduziert wird und die Atemluft nicht außen an dem Kanülenrohr vorbei geleitet werden muss, was unter Umständen schon durch einen die Kanüle innerhalb der Trachea umgebenden und fixierenden Cuff verhindert wird, ist in der Wand des Kanülenrohrs zwischen dem distalen Ende und dem proximalen Ende die bereits erwähnte Phonationsöffnung angeordnet. Beim Ausatmen kann die Atemluft zunächst durch die distale Öffnung in das Kanülenrohr hinein und durch die Phonationsöffnung wieder heraus und in die oberen, natürlichen Atemwege nach außen gelangen. Auch beim Einatmen könnte die Luft, soweit sie nicht durch die proximale Öffnung bzw. durch ein Sprechventil kommt, von den oberen, natürlichen Atemwegen durch die Phonationsöffnung in das Kanülenrohr strömen. Bei Tracheostomiekanülen mit einem Cuff, der den Spalt zwischen Trachealwand und Kanüle verschließt, bietet die Phonationsöffnung bei der Verwendung eines Sprechventils den einzigen Strömungsweg, durch den die Atemluft ausgeatmet werden kann.

Da Tracheostomiekanülen nur in einer begrenzten Anzahl verschiedener Größen verfügbar sind und auch aufgrund von anatomischen Besonderheiten vieler Patienten ist die jeweils verwendete Tracheostomiekanüle oft nicht optimal an Größe des Patienten bzw. dessen Trachea angepasst. Dadurch kommt es nicht selten vor, dass die Phonationsöffnungen von Tracheostomiekanülen ganz oder teilweise im Bereich des Tracheostoma liegen. Dies erhöht den Atemwiderstand, sodass die Phonation stark eingeschränkt oder verhindert wird. In schweren Fällen besteht Erstickungsgefahr. Solange das Stoma nicht verheilt ist, besteht zusätzlich ein Emphysemrisiko. Bei einem Emphysem dringt Luft, beispielsweise aus der Phonationsöffnung herkömmlicher Tracheostomiekanülen, in das das Tracheostoma umgebende Gewebe ein und bläht dieses auf. Die an einem Emphysem leidenden Patienten wirken aufgedunsen und fühlen sich unwohl. Auch erhöht die in das Gewebe eindringende Luft das Infektionsrisiko.

Bei der Verwendung herkömmlicher Tracheostomiekanülen mit Phonationsöffnung kann zudem ein zum Reinigen in das Kanülenrohr durch die proximale Öffnung eingeführter Absaugschlauch versehentlich durch die Phonationsöffnung in die Trachea gelangen und die Trachealwand verletzen.

Weiterhin kann auch der Rand der Phonationsöffnung herkömmlicher Tracheostomiekanülen an der Trachealwand reiben und die Trachealwand verletzen. Das sich infolge einer Verletzung der Trachealwand oder auch innerhalb des Tracheostomas bildende Granulationsgewebe kann in die Phonationsöffnung einwachsen. Beim Herausziehen oder Bewegen des Kanülenrohrs kann es durch das gewaltsame Lösen des eingewachsenen Granulationsgewebes zu Schmerzen, Verletzungen der Trachea und Komplikationen, wie zum Beispiel Blutungen, kommen. Ein Einwachsen von Granulationsgeweben in die Phonationsöffnung wird häufiger beobachtet, wenn die Tracheostomiekanüle nicht optimal an die Trachea des Patienten angepasst ist.

Nachfolgende Dokumente offenbaren bereits bekannte Tracheostomiekanülen GB 2 340 401, US 4 459 984, EP 2 801 384, WO 2016/038323, US 6 722 367, EP 2 322 240, EP 2 810 618, DE 10 2016 002841, JP 2016 026542, US 4 280 492, WO 99/32169.

Trotz anerkannter Vorteile einer Phonationsöffnung, verzichten viele Anwender daher auf Tracheostomiekanülen mit Phonationsöffnungen und die betroffenen Patienten verlieren viel Lebensqualität aufgrund der fehlenden bzw. eingeschränkten Möglichkeit, sich über Sprache zu äußern.

Es ist daher eine Aufgabe der vorliegenden Erfindung eine Tracheostomiekanüle bereitzustellen, die die Gefahr einer Verletzung der Trachealwand und/oder des Einwachsens von Granulationsgewebe reduziert und dadurch die Verwendung von Tracheostomiekanülen mit Phonationsöffnung erleichtert.

Diese Aufgabe wird durch die erfindungsgemäße Vorrichtung nach Anspruch 1 gelöst. Diese Aufgabe wird mindestens teilweise durch die eingangs beschriebene Tracheostomiekanüle gelöst, wobei eine Abdeckung mit mindestens einem permanent geöffneten Luftdurchlass den Öffnungsquerschnitt der Phonationsöffnung überspannt.

Die Abdeckung verhindert, dass der Rand der Phonationsöffnung an der Trachealwand reibt und/oder sich ein Teil der Trachealwand oder des Tracheostomagewebes in die Phonationsöffnung hineinerstreckt. Insbesondere an der trachealen Hinterwand (aus Sicht des Patienten) können mit Hilfe der Abdeckung vermindert werden. Dadurch werden Verletzungen durch den Rand der Phonationsöffnung vermieden und es kommt zu keiner Bildung von Granulationsgewebe, welches in die Phonationsöffnung einwachsen könnte. Zudem bildet die Abdeckung eine mechanische Barriere, die verhindert, dass ein durch die proximale Öffnung in das Kanülenrohr geführter Absaugschlauch versehentlich durch die Phonationsöffnung in die Trachea hindurchgestoßen und die Trachealwand verletzt wird.

Obwohl die Abdeckung die Phonationsöffnung überspannt, verschließt sie die Phonationsöffnung nicht vollständig. Der mindestens eine permanent geöffnete Luftdurchlass ermöglicht, dass bei der Verwendung der Tracheostomiekanüle jederzeit Luft durch die Phonationsöffnung hindurch strömen kann. Durch den dauerhaft geöffneten Luftdurchlass kann Luft aus den oberen, natürlichen Atemwegen beim Einatmen durch die Phonationsöffnung in das Kanülenrohr oder beim Ausatmen aus dem Kanülenrohr durch die Phonationsöffnung und den Luftdurchlass in die oberen, natürlichen Atemwege gelangen. Ein vollständiger Verschluss der proximalen Öffnung des Kanülenrohrs oder die Verwendung eines Sprechventils an der proximalen Öffnung des Kanülenrohrs führt somit nicht zu einer akuten Lebensgefahr für den Patienten, da Atemluft nach wie vor durch die nicht vollständig verschlossene Phonationsöffnung, die distale Öffnung des Kanülenrohrs und die natürlichen Atemwege ein- und ausströmen kann.

Die Abdeckung ist erfindungsgemäß mindestens so groß wie der Öffnungsquerschnitt der Phonationsöffnung und überspannt den Öffnungsquerschnitt der Phonationsöffnung vollständig.

Die Abdeckung ist vorzugsweise auf der Außenseite des Kanülenrohrs angeordnet.

Die Begriffe "distal" und "proximal" werden im Sinne der vorliegenden Erfindung aus der Sicht eines behandelnden Arztes verwendet, d.h. das proximale Ende der Tracheostomiekanüle bzw. des Kanülenrohrs liegt außerhalb des Patientenkörpers, während das distale Ende im eingebrachten Zustand der Tracheostomiekanüle im Inneren der Trachea des Patienten angeordnet ist.

In einer Ausführungsform weist die Abdeckung einen umlaufenden, sich nach dem Aufbringen der Abdeckung über den Rand der Phonationsöffnung hinaus erstreckenden Überstand auf, wobei der Überstand vorzugsweise mindestens 1 mm beträgt. Der Überstand überlappt mit dem die Phonationsöffnung umgebenden Wandabschnitt des Kanülenrohrs und kann zur Befestigung der Abdeckung an dem Kanülenrohr verwendet werden.

In einer Ausführungsform ist die Abdeckung derart in eine die Phonationsöffnung umgebende Vertiefung der Kanülenwand eingelassen, dass die Abdeckung bündig mit der Außenfläche der Wand des Kanülenrohrs abschließt oder vollständig in der Vertiefung in der Wand des Kanülenrohrs angeordnet ist. Eine bündig mit der Außenfläche der Kanülenwand abschließende oder vollständig in einer Vertiefung in der Wand des Kanülenrohrs angeordnete Abdeckung steht in einer Querschnittsansicht durch das Kanülenrohr nicht über dessen äußere Oberfläche hervor. Das Kanülenrohr kann somit in eine Trachea ein- und ausgeführt werden, ohne dass die Abdeckung an die Innenwand der Trachea anstößt oder an dieser reibt.

In einer Ausführungsform ist die Abdeckung derart in der Phonationsöffnung angeordnet, dass sie von einem ersten Zustand, in dem die Abdeckung nicht über die Außenfläche der Wand des Kanülenrohrs hervorsteht und/oder in dem die Abdeckung teilweise in das Lumen des Kanülenrohrs hineinragt, in einen zweiten Zustand, in dem die Abdeckung über die Außenfläche der Wand des Kanülenrohrs hervorsteht, bringbar ist. Zum Einführen der Tracheostomiekanüle ist es von Vorteil, wenn die Abdeckung vorzugsweise keine über die Außenfläche der Wand des Kanülenrohrs hervorstehenden Abschnitte aufweist, auch wenn sie dann womöglich teilweise in das Lumen des Kanülenrohrs hineinragt. Auf diese Weise kann die Tracheostomiekanüle einfach und ohne dass die Abdeckung an der Trachealwand reibt, in die Trachea eingeführt werden.

Nach dem Platzieren in der Trachea kann die Abdeckung beispielsweise durch Einführen einer Innenkanüle in das Kanülenrohr in den zweiten Zustand überführt werden. Die Innenkanüle drückt dann den zuvor in dem ersten Zustand der Abdeckung die teilweise in das Lumen des Kanülenrohrs hineinragende Abdeckung nach Außen, sodass sie in ihrem zweiten Zustand zumindest teilweise über die Außenfläche des Kanülenrohrs hervorsteht. Um das Überführen der Abdeckung aus dem ersten Zustand in den zweiten Zustand zu erleichtern, kann die Abdeckung auf ihrer dem Lumen des Kanülenrohrs zugewandten Seite eine Gleitschiene zum in Eingriff bringen mit einer durch das Kanülenrohr einführbaren Innenkanüle aufweisen. Die Gleitschiene kann mit oder ohne Fase ausgestaltet sein. Eine Gleitschiene mit Fase hilft ein versehentliches Einklemmen der Abdeckung zwischen der Außenfläche einer Innenkanüle und der Innenfläche des Kanülenrohrs zu verhindern.

Die in der Phonationsöffnung angeordnete Abdeckung kann für den ersten Zustand oder den zweiten Zustand und auch bistabil vorgespannt sein, sodass die Abdeckung das Bestreben hat, zumindest nach Überschreiten eines Totpunktes selbststätig in einen der Zustände überzugehen. Alternativ kann die Abdeckung beispielsweise nach dem Entfernen einer Innenkanüle, die die Abdeckung in den zweiten Zustand hält, selbsttätig in den ersten Zustand zurückkehren. Auch kann eine Innenkanüle mit Phonationsöffnung vorgesehen sein, wobei die Abdeckung und die Innenkanüle mit Phonationsöffnung derart aufeinander abgestimmt sind, dass beim Einführen der Innenkanüle mit Phonationsöffnung die Abdeckung in den zweiten Zustand überführt wird. Beispielsweise können die Abdeckung und die Innenkanüle mit Phonationsöffnung aufeinander abgestimmte Eingriffsmittel, wie z.B. Vorsprünge, Gleitschienen oder Gleitflächen, aufweisen, sodass die Abdeckung durch das in Eingriff bringen der Eingriffsmittel in den zweiten Zustand überführt wird. Das Einsetzen einer Innenkanüle ohne die entsprechenden Eingriffsmittel führt dagegen nicht zu einem Übergang der Abdeckung in den zweiten Zustand. Vorzugsweise sind die Eingriffsmittel der Innenkanüle im Bereich der Phonationsöffnung der Innenkanüle angeordnet oder sind Teil des Randes der Phonationsöffnung, sodass eine Innenkanüle ohne Phonationsfenster die Abdeckung nicht in den zweiten Zustand überführt.

Erfindungsgemäß hält ein die Phonationsöffnung zumindest abschnittsweise umgebender Vorsprung die Abdeckung auf Abstand zu der Kanülenwand, wobei der Vorsprung den mindestens einen permanent geöffneten Luftdurchlass bereitstellt bzw. bewirkt. Der Vorsprung hält die Abdeckung in einem vorbestimmten Abstand zu dem Kanülenrohr. Eine Unterbrechung des Vorsprunges oder eine Aussparung in dem Vorsprung kann den permanent geöffneten Luftdurchlass bilden. Da der Luftdurchlass in dem Vorsprung angeordnet ist, wird er nicht durch Anlage an der Trachea oder Tracheostomagewebe abgedeckt.

In einer Ausführungsform weist der Vorsprung eine Mehrzahl von permanent geöffneten Luftdurchlässen, vorzugsweise zwischen zwei und zehn Luftdurchlässe, auf, die entlang des Umfangs der Phonationsöffnung verteilt, vorzugsweise gleichmäßig und paarweise diametral zueinander, angeordnet sind. Die Mehrzahl von Luftdurchlässen ermöglicht es, den permanent offenen Gesamtöffnungsquerschnitt auf mehrere Luftdurchlässe aufzuteilen, sodass der Öffnungsquerschnitt jedes einzelnen Luftdurchlasses kleiner ausgeführt werden kann. Kleinere Öffnungsquerschnitte reduzieren das Risiko, dass Gewebe in die Luftdurchlässe des Vorsprunges einwächst, zumal eine gedachte Durchströmungsachse dieser Öffnungen sich nicht in radialer Richtung erstreckt, sondern in axialer Richtung der Kanüle parallel zu deren Außenwand verläuft.

Entlang des Umfangs der Phonationsöffnung verteilt angeordnete Luftdurchlässe erlauben einen gleichmäßigen Luftstrom und reduzieren die Gefahr, dass alle Luftdurchlässe gleichzeitig durch ein unbeabsichtigtes Anlegen der Abdeckung an eine Seite der Trachea verschlossen werden.

Ähnlich wie im Falle einer Abdeckung, die entsprechende Vorsprünge nicht aufweist, ist der gesamte, den oder die Vorsprünge aufweisende Rand der Phonationsöffnung zusammen mit der Abdeckung bezüglich der Kanüle radial beweglich in einen ersten Zustand, in welchem die Abdeckung mit der Außenseite der Kanüle bündig abschließt oder radial eingezogen ist, und in einen zweiten Zustand in welchem der oder die Vorsprünge zusammen mit der darauf angeordnete Abdeckung über die Außenfläche der Kanüle hervorsteht. Der Rand könnte beispielsweise aus einem entsprechend elastischem Material bestehen und bistabil mit einem zwischen den erwähnten Zuständen liegenden Totpunkt vorgespannt sein.

In einer Ausführungsform sind die Luftdurchlässe auf der distalen und/oder proximalen Seite der Phonationsöffnung und/oder lateral von dieser angeordnet. Distale und/oder proximale Luftdurchlässe erlauben einen Luftstrom der überwiegend in die distale bzw. proximale Richtung des Kanülenrohrs strömt. Lateral von der Phonationsöffnung angeordnete Luftdurchlässe sind insbesondere beim Ein- und Ausführen des Kanülenrohrs in die Trachea besser gegen das Eindringen oder Zusetzen durch Sekret geschützt als distale oder proximale Luftdurchlässe.

In einer Ausführungsform können die vorgenannten Vorsprünge auch die Form einer Profilierung auf der der Kanülenwand zugewandten Seite der Abdeckung haben, die sich über den Öffnungsquerschnitt der Phonationsöffnung erstreckt und mit dem Kanülenrohr in Eingriff steht. Die Profilierung dient als Abstandhalter und verhindert, dass die Abdeckung großflächig an der die Phonationsöffnung umgebenden Wand des Kanülenrohrs anliegt und die Phonationsöffnung vollständig verschließt. Gleichzeitig stabilisiert die Profilierung die Abdeckung und verhindert, dass die Abdeckung sich durch ihr Eigengewicht durchbiegt bzw. durchhängt. Die Profilierung kann die Form von sich über den Öffnungsquerschnitt erstreckenden Stegen aufweisen, die an der Wand des Kanülenrohrs anliegen bzw. mit dieser in Eingriff stehen. Durch zwischen den Stegen gebildete Nuten kann Atemluft in die Phonationsöffnung ein- und ausströmen. Die Nuten bilden permanent geöffnete Verbindungen zu dem Phonationsfenster.

In einer Ausführungsform ist die Abdeckung mit dem Kanülenrohr verklebt, verschweißt und/oder angeformt. Es versteht sich, dass die zuvor genannte Profilierung und/oder der zuvor genannte Vorsprung sowohl mit dem Kanülenrohr als auch mit der Abdeckung verklebt, verschweißt und/oder angeformt sein kann, sodass letztendlich die Abdeckung auch über die Profilierung und/oder einen oder mehrere Vorsprünge an dem Kanülenrohr befestigt sein kann.

In einer weiteren Ausführungsform ist die Abdeckung nur stellenweise, vorzugsweise neben dem distalen und/oder proximalen Ende der Phonationsöffnung an dem Kanülenrohr befestigt. Eine nur stellenweise an dem Kanülenrohr befestigte Abdeckung kann sich beim dem durch Atmung in dem Kanülenrohr erzeugten Druck aufwölben bzw. von der Phonationsöffnung weiter abgehoben werden. Auf diese Weise wird der Öffnungsquerschnitt der Phonationsöffnung beim Ausatmen vergrößert. Der Atemwiderstand beim Ausatmen verringert sich. Vorzugsweise hat die Abdeckung eine Shore-A-Härte zwischen 5 und 90, besonders bevorzugt zwischen 20 und 60.

In einer Ausführungsform weist die Abdeckung einen permanent geöffneten Luftdurchlass in Form einer schlitzförmigen Durchbrechung auf, die vorzugsweise in einem Bereich radial außerhalb des Kanülenrohrprofils oder bündig mit diesem angeordnet ist. Die schlitzförmige Durchbrechung bildet eine permanent geöffnete, d.h. zu jeder Zeit von Atemluft durchströmbare Öffnung. Die schlitzförmige Durchbrechung ist fluchtend, mit dem äußeren Rand der Phonationsöffnung angeordnet. Beim Ein- und Ausatmen weitet sich die schlitzförmige Durchbrechung durch den Atemdruck und vergrößert so den Öffnungsquerschnitt. Vorzugsweise weist die Abdeckung einen permanent geöffneten Luftdurchlass in Form zweier sich kreuzender Schlitze auf. Kreuzen sich zwei oder mehr schlitzförmige Durchbrechungen bilden sich die an die kreuzenden Durchbrechungen angrenzenden Abschnitte der Abdeckung Flügel oder Segelabschnitte, die durch den Atemdruck aufgebogen werden und sich elastisch schließen können.

In einer Ausführungsform weist das Kanülenrohr einen Cuff mit einem proximalen Halsabschnitt auf, wobei der Halsabschnitt den Cuff in Umfangsrichtung des Kanülenrohrs abdichtet und die Abdeckung Teil des Halsabschnittes ist. Der Halsabschnitt und die Abdeckung sind somit einteilig ausgeführt, wobei die Abdeckung beim Anbringen des Cuffs über der Phonationsöffnung angeordnet wird. Der Cuff und das Kanülenrohr sind getrennt voneinander herstellbar und können nach der getrennten Herstellung zusammengefügt werden. Auf diese Weise ist die Herstellung der Tracheostomiekanüle insgesamt einfacher.

Ein Cuff besteht üblicherweise aus einem aufblasbaren, sehr dünnwandigen Schlauch, dessen Endabschnitte außen auf dem Kanülenrohr eng anliegen und dort befestigt sind und der mindestens in einem zentralen Abschnitt gegenüber dem Kanülenrohr ein deutliches Übermaß hat oder auf ein solches Übermaß aufblasbar ist, so dass dieser Abschnitt sich nach dem Einführen der Kanüle in die Trachea aufblasen lässt und mit der Wand der Trachea entlang seines Umfangs abdichtend und mit vorzugsweise geringem Druck in Kontakt kommt. Die Endabschnitte, die im Allgemeinen ihrerseits Schlauchabschnitte (hier auch "Halsabschnitte" genannt) darstellen, sind typischerweise über ihre Länge und ihren Umfang hinweg mit der Außenwand des Kanülenrohres dicht verklebt oder verschweißt. Der axial äußere Rand des proximalen Endabschnittes bildet dann den vorstehend definierten Bereich für eine bevorzugte Anordnung einer Abdeckung.

In einer Ausführungsform besteht die Abdeckung aus einem elastischen Material, vorzugsweise einer elastischen Folie, mit einer Shore-A-Härte von einschließlich 5 bis einschließlich 90, vorzugsweise mit einer Shore-A-Härte von einschließlich 20 bis einschließlich 60. Das elastische Material oder die elastische Folie sind dafür geeignet durch den Atemdruck reversibel verformt zu verwenden. Auf diese Weise kann der Öffnungsquerschnitt beim Ein- und Ausströmen von Atemluft vorübergehend vergrößert werden. Der Atemwiderstand wird reduziert. Strömt keine Atemluft durch die Phonationsöffnung kehrt die Abdeckung in ihre weitgehend geschlossene Ausgangslage zurück, wobei der mindestens der Luftdurchlass sich jederzeit bei auftretenden Druckdifferenzen zwischen Innen- und Außenseite des Kanülenrohrs öffnen kann.

In einer Ausführungsform hat die Abdeckung eine Wandstärke von maximal 2 mm, vorzugsweise von maximal 1 mm, wobei die Wandstärke vorzugsweise mindestens 0,01 mm beträgt. Ein Material mit einer Materialstärke und einer Shore Härte in dem beanspruchten Bereich ist gleichermaßen widerstandsfähig und flexibel und eignet sich daher besonders gut für die Herstellung der Abdeckung.

In einer Ausführungsform ist die Phonationsöffnung ein Phonationsfenster oder ein Phonationssieb. Bei einem Phonationsfenster wird der Öffnungsquerschnitt der Phonationsöffnung durch eine einzelne Öffnung gebildet, während sich der Öffnungsquerschnitt der Phonationsöffnung bei einem Phonationssieb auf mehrere Öffnungen verteilt. Ein Phonationssieb hat den Vorteil, dass beispielsweise ein durch die proximale Öffnung in das Kanülenrohr eingebrachter Absaugschlauch nicht versehentlich durch die Phonationsöffnung in die Trachea gelangen kann.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1: eine schematische Perspektivansicht einer Tracheostomiekanüle gemäß der vorliegenden Erfindung,
- Figur 2: eine Detailansicht einer Abdeckung für eine Tracheostomiekanüle gemäß der vorliegenden Erfindung,
- Figur 3: eine Detailansicht einer Abdeckung für eine Tracheostomiekanüle gemäß einer Ausführungsform der vorliegenden Erfindung,
- Figur 4: eine schematische Perspektivansicht einer Tracheostomiekanüle gemäß einer weiteren Ausführungsform der vorliegenden Erfindung,
- Figur 5: eine schematische Perspektivansicht einer Tracheostomiekanüle gemäß noch einer weiteren Ausführungsform der vorliegenden Erfindung,
- Figur 6: eine Tracheostomiekanüle mit einer Phonationsöffnung gemäß der vorliegenden Erfindung, und
- Figur 7: eine Tracheostomiekanüle mit einer Phonationsöffnung gemäß der vorliegenden Erfindung.

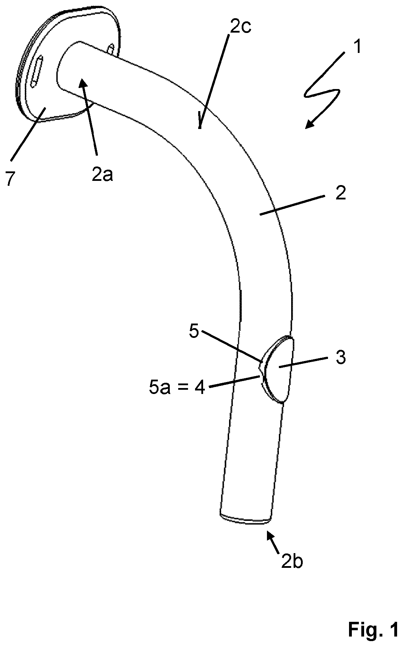

In der Figur 1 ist eine Tracheostomiekanüle 1 gemäß der vorliegenden Erfindung gezeigt. Die Tracheostomiekanüle 1 hat ein Kanülenrohr 2 mit einem proximalen Ende 2a und einem distalen Ende 2b sowie eine Wand 2c des Kanülenrohres 2. Das proximale Ende 2a und das distale Ende 2b weisen jeweils eine Öffnung auf. Im Betrieb der Tracheostomiekanüle 1 ist das distale Ende 2b mit der distalen Öffnung in der Trachea eines Patienten und das proximale Ende 2a mit der proximalen Öffnung außerhalb des Patienten angeordnet. Auf diese Weise kann ein Patient durch die Tracheostomiekanüle 1 selbsttätig atmen oder beatmet werden.

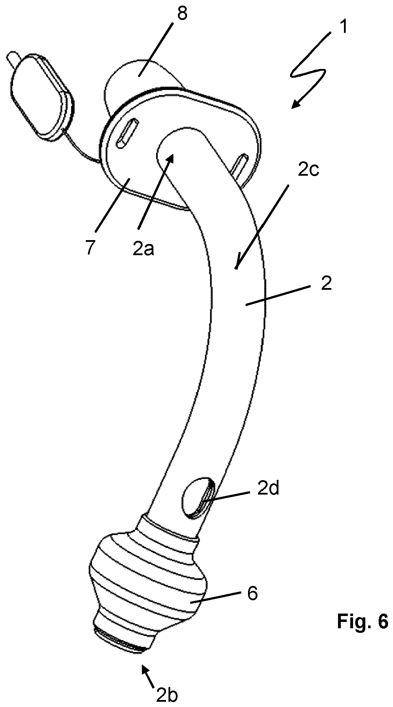

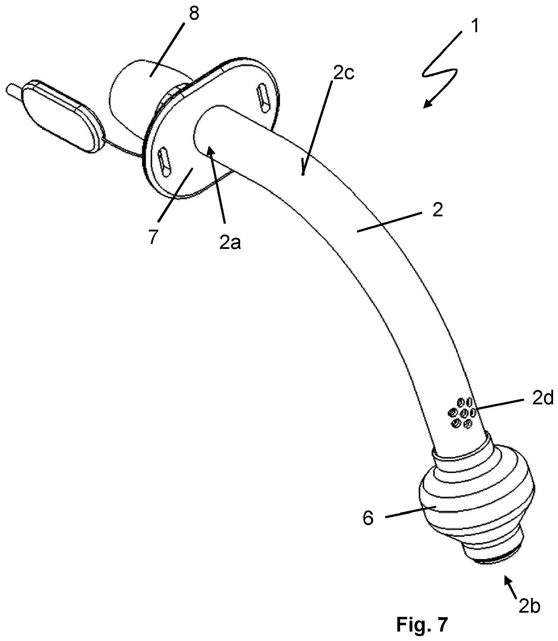

Zum Fixieren der Tracheostomiekanüle 1 am Hals eines Patienten ist an dem proximalen Ende 2a des Kanülenrohrs ein Kanülenschild 7 angeordnet. An dem Kanülenschild 7 kann ein Band befestigt werden, welches um den Hals des Patienten gelegt wird und so die Tracheostomiekanüle fixiert. Um das Sprechen für einen Patienten mit einer in die Trachea eingesetzten Tracheostomiekanüle 1 zu ermöglichen, ist in der Wand 2c des Kanülenrohrs eine Phonationsöffnung 2d, wie sie beispielhaft in den Figuren 6 und 7 dargestellt ist, zwischen dem proximalen Ende 2a und dem distalen Ende 2b an einer Position vorgesehen, die bei eingesetzter Tracheostomiekanüle innerhalb der Trachea des Patienten liegt. Atemluft kann demnach beim Ausatmen durch die distale Öffnung in das Kanülenrohr 2 und durch die Phonationsöffnung 2d in die Trachea und durch die natürlichen Atemwege an den Stimmbändern vorbeiströmen, wenn die proximale Öffnung gegen ausströmende Atemluft gesperrt ist.

Auf der Außenseite des Kanülenrohrs 2 ist eine Abdeckung 3 angeordnet, die den Öffnungsquerschnitt der Phonationsöffnung 2d überspannt. Die Abdeckung 3 ist etwas größer als der Öffnungsquerschnitt der Phonationsöffnung 2d und erstreckt sich über den Rand der Phonationsöffnung 2d hinaus. Der den Rand der Phonationsöffnung überlappende Überstand der Abdeckung beträgt ca. 1 mm und ist entlang des gesamten Umfangs der Phonationsöffnung 2d vorgesehen.

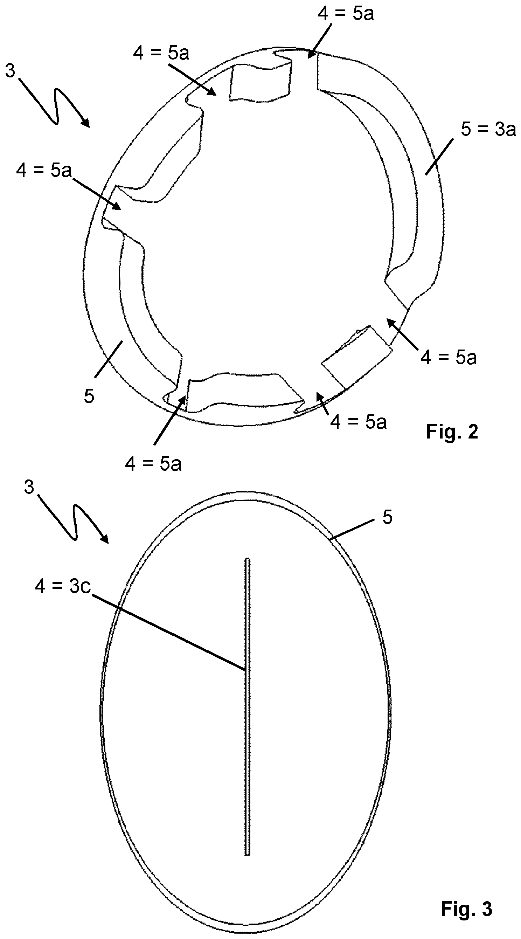

Ein unterbrochener, die Phonationsöffnung 2d umgebender Vorsprung 5 dient als Auflage der Abdeckung auf einem die Phonationsöffnung umgebenden Rand. Der Vorsprung ist vorzugsweise einstückig mit dem überstehenden Rand 3a der Abdeckung 3 (siehe Figur 2) ausgebildet und zumindest Teile des Vorsprungs sind, z, B. durch Verkleben, mit der Wand 2c des Kanülenrohrs 2 verbunden. Die Abdeckung steht radial über die Außenfläche des Kanülenrohrs hervor und die Unterbrechungen 5a des Vorsprunges 5 bilden jeweils einen permanent geöffneten Luftdurchlass 4, durch den Atemluft durch die Phonationsöffnung 2d ein- und ausströmen kann.

In der Figur 2 ist eine solche Abdeckung 3 für eine Tracheostomiekanüle 1 gemäß der vorliegenden Erfindung in einer Detailansicht der der Wand 2c des Kanülenrohrs zugewandten Seite der Abdeckung 3 gezeigt. Gut erkennbar sind entlang des überstehenden Randes der Abdeckung 3 umlaufende Vorsprungsabschnitte, die als Abstandhalter für die Abdeckung 3 dienen und die individuell jeweils als Vorsprung oder gemeinsam auch als ein mehrfach unterbrochener Vorsprung angesehen werden könnten. Der Vorsprung 5 dient gleichzeitig der Verbindung der Abdeckung mit dem Kanülenrohr 2 verbindet. Der Vorsprung 5 weist sechs Unterbrechungen 5a auf, die jeweils einen permanent geöffneten Luftdurchlass 4 bilden durch den Luft ein- bzw. ausströmen kann. Die Luftdurchlässe 4 sind paarweise diametral gegenüberliegend zueinander angeordnet.

Eine alternative Ausgestaltung einer Abdeckung 3 ist in der Figur 3 dargestellt. Die Abdeckung 3 weist eine schlitzförmige Durchbrechung 3c auf, die gemäß einer Ausführungsform der vorliegenden Erfindung radial außenliegend über der Phonationsöffnung 2d einer Tracheostomiekanüle 1 angeordnet ist. Die schlitzförmige Durchbrechung 3c ist permanent geöffnet bzw. öffnet sich jederzeit bei einem Druckunterschied zwischen dem Inneren und Äußeren des Kanülenrohres 2, sodass jederzeit Atemluft durch die Durchbrechung 3c und die Phonationsöffnung 2d strömen kann. Die schlitzförmige Durchbrechung bildet einen permanent geöffneten Luftdurchlass.

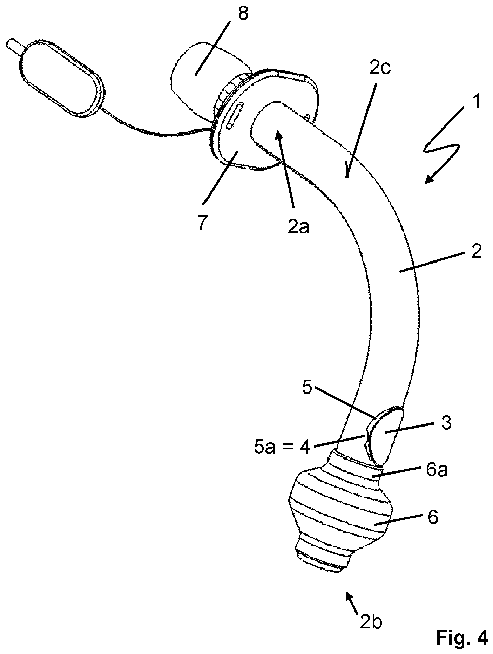

In Figur 4 ist eine Tracheostomiekanüle 1 gemäß einer weiteren Ausführungsform der vorliegenden Erfindung dargestellt. Wie in den anderen Ausführungsformen hat die Tracheostomiekanüle 1 ein Kanülenrohr 2, welches ein proximales Ende 2a und ein distales Ende 2b aufweist. An dem proximalen Ende 2a befindet sich ein Kanülenschild 7 und ein Konnektor 8 zum Anschluss an eine Beatmungsvorrichtung.

An dem distalen Ende 2a ist ein mit Fluid befüllbarer Cuff 6 angeordnet. Der Cuff 6 ist außerhalb des Kanülenrohrs 2 angeordnet und weist zwei Halsabschnitte auf, die eng an dem Kanülenrohr 2 anliegen und das befüllbare Volumen des Cuffs gegenüber dem Kanülenrohr 2 abdichten. Wird der Cuff 6 mit einem Fluid befüllt, dehnt er sich auf ein Übermaß gegenüber dem Kanülenrohr 2 aus und legt sich vollumfänglich an die Innenwand einer Trachea an, in die das Kanülenrohr 2 eingeführt ist.

Proximal von dem Cuff 6 befindet sich eine Abdeckung 3, die eine dort in der Wand 2c des Kanülenrohrs 2 angeordnete Phonationsöffnung 2d überspannt. Die Abdeckung 3 entspricht der in den Figuren 1 und 2 beschriebenen Ausführungsform.

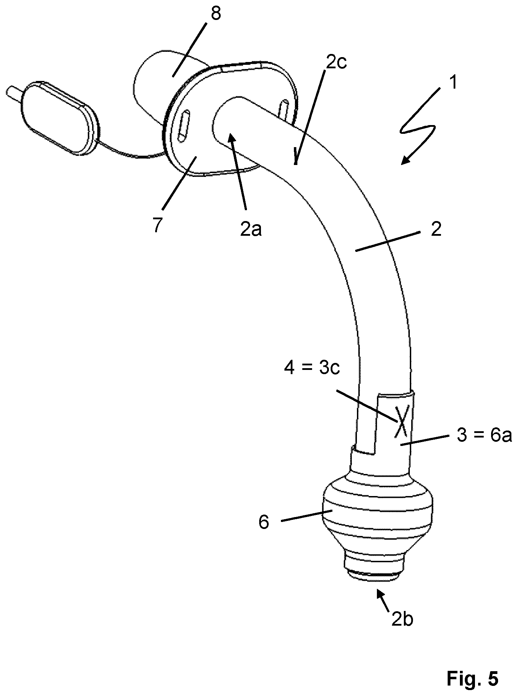

Die in der Figur 5 gezeigte Ausführungsform unterscheidet sich von der in der Figur 4 gezeigten Ausführungsform in der Ausgestaltung der Abdeckung 3. Bei der in der Figur 5 gezeigten Ausführungsform ist die Abdeckung 3 Teil des proximalen Halsabschnittes 6a des Cuffs 6. Der proximale Halsabschnitt 6a erstreckt sich außerhalb des Kanülenrohrs 2 über die in der Wand 2c des Kanülenrohrs befindliche Phonationsöffnung 2d. Der proximale Halsabschnitt 6a ist aus einem elastischen Material mit einer Shore-A-Härte von einschließlich 5 bis einschließlich 90 gefertigt. In dem die Phonationsöffnung 2d überdeckenden Bereich der Abdeckung 3 sind zwei sich kreuzende Schlitze 3c angeordnet, die elastisch biegsame dreieckige Flügel voneinander trennen und permanent geöffnete bzw. jederzeit zu öffnende Luftdurchlässe bilden. Das elastische Material der durch den proximalen Halsabschnitt 6a gebildeten Abdeckung 3 führt dazu, dass sich der Öffnungsquerschnitt der sich kreuzenden Schlitze durch elastisches Wegbiegen der Flügel unter dem Druck ausströmender Atemluft vergrößert und so den Atmungswiderstand reduziert, wenn die Luft durch die Phonationsöffnung aus dem Kanülenrohr 2 in die äußeren Atemwege strömt.

In den Figuren 6 und 7 ist eine erfindungsgemäße Tracheostomiekanüle 1 zur Verdeutlichung der Phonationsöffnung 2d ohne eine Abdeckung 3 dargestellt. Die Phonationsöffnung 2d kann, wie in der Figur 7 dargestellt, auch in Form einer Siebung vorliegen.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Tracheostomiekanüle
- 2: Kanülenrohr
- 2a: proximale Ende des Kanülenrohrs
- 2b: distale Ende des Kanülenrohrs
- 2c: Kanülenwand
- 2d: Phonationsöffnung
- 3: Abdeckung
- 3a: Überstand
- 3c: schlitzförmige Durchbrechung
- 4: Luftdurchlass
- 5: Vorsprung
- 5a: Unterbrechung
- 6: Cuff
- 6a: proximaler Halsabschnitt des Cuffs
- 7: Kanülenschild
- 8: Konnektor

## Patentansprüche

1. Tracheostomiekanüle (1) mit einem Kanülenrohr (2), wobei das Kanülenrohr (2) ein proximales Ende (2a) mit einer proximalen Öffnung, ein distales Ende (2b) mit einer distalen Öffnung, sowie eine Phonationsöffnung (2d) zwischen dem proximalen Ende (2a) und dem distalen Ende (2b) in der Wand des Kanülenrohrs (2) aufweist, wobei eine Abdeckung (3) den Öffnungsquerschnitt der Phonationsöffnung (2d) vollständig überspannt und mindestens einen bei der Verwendung der Tracheostomiekanüle permanent geöffneten Luftdurchlass (4) aufweist, sodass jederzeit Luft aus den oberen, natürlichen Atemwegen beim Einatmen durch die Phonationsöffnung in das Kanülenrohr oder beim Ausatmen aus dem Kanülenrohr durch die Phonationsöffnung und den Luftdurchlass in die oberen, natürlichen Atemwege gelangen kann, wobei ein entlang des äußeren Randes der Abdeckung (3) zumindest abschnittsweise umlaufender Vorsprung (5) als Abstandhalter gegenüber der Außenseite des Kanülenrohrs (2) dient und mindestens teilweise mit dem Kanülenrohr (2) verbunden ist, wobei der Vorsprung (5) den mindestens einen permanent geöffneten Luftdurchlass (4) aufweist.

2. Tracheostomiekanüle (1) nach Anspruch 1, **dadurch gekennzeichnet, dass** die Abdeckung (3) derart in eine die Phonationsöffnung (2d) umgebende Vertiefung (2e) in der Wand (2c) des Kanülenrohrs (2) eingelassen ist, dass die Abdeckung (3) zumindest distal bündig mit der Außenfläche des Kanülenrohrs (2) abschließt oder vollständig in der Vertiefung (2e) in der Wand (2c) des Kanülenrohrs (2) angeordnet ist.

3. Tracheostomiekanüle (1) nach einem der Ansprüche 1 oder 2, **dadurch gekennzeichnet, dass** die Abdeckung (3) derart in der Phonationsöffnung (2d) angeordnet ist, dass die Abdeckung (3) von einem ersten Zustand, in dem die Abdeckung (3) nicht über die Außenfläche der Wand (2c) des Kanülenrohrs (2) hervorsteht und/oder in dem die Abdeckung (3) teilweise in das Lumen des Kanülenrohrs (2) hineinragt, in einen zweiten Zustand, in dem die Abdeckung (3) über die Außenfläche der Wand (2c) des Kanülenrohrs (2) hervorsteht, bringbar ist.

4. Tracheostomiekanüle (1) nach Anspruch 3, **dadurch gekennzeichnet, dass** eine Innenkanüle mit einer Phonationsöffnung in das Kanülenrohr (2) eingesetzt ist, wobei die Abdeckung (3) und die Innenkanüle mit Phonationsöffnung derart aufeinander abgestimmt sind, dass die Abdeckung (3) durch das Einsetzen der Innenkanüle mit Phonationsöffnung in das Kanülenrohr (2) von dem ersten Zustand in den zweiten Zustand überführbar ist.

5. Tracheostomiekanüle (1) nach einem der Ansprüche 1 bis 4, **dadurch gekennzeichnet, dass** der Vorsprung (5) aus mehreren Vorsprungsabschnitten besteht, die durch eine Mehrzahl, vorzugsweise 2 bis 10, permanent geöffneter Luftdurchlässe (4) getrennt sind, die entlang des Umfangs der Phonationsöffnung (2d), vorzugsweise gleichmäßig verteilt und paarweise diametral zueinander, angeordnet sind.

6. Tracheostomiekanüle (1) nach einem der Ansprüche 1 bis 5, **dadurch gekennzeichnet, dass** die Abdeckung (3) auf ihrer der Wand (2c) des Kanülenrohrs zugewandten Seite eine Profilierung aufweist, die sich vom äußeren Umfang der Abdeckung über den Rand der Phonationsöffnung (2d) hinweg erstreckt und mit dem Kanülenrohr (2) in Eingriff steht.

7. Tracheostomiekanüle (1) nach einem der Ansprüche 1 bis 6, **dadurch gekennzeichnet, dass** die Abdeckung (3) mit dem Kanülenrohr (2) verklebt, verschweißt und/oder an dieses angeformt ist.

8. Tracheostomiekanüle (1) nach einem der Ansprüche 1 bis 7, **dadurch gekennzeichnet, dass** die Abdeckung (3) entlang ihres Umfangs nur an voneinander beabstandeten Stellen, vorzugsweise auf der distalen und/oder proximalen Seite der Phonationsöffnung (2d), an dem Kanülenrohr (2) befestigt ist.

9. Tracheostomiekanüle (1) nach einem der Ansprüche 1 bis 8, **dadurch gekennzeichnet, dass** die Abdeckung (3) mindestens einen permanent geöffneten Luftdurchlass (4) in Form einer schlitzförmigen Durchbrechung (3c) aufweist.

10. Tracheostomiekanüle (1) nach Anspruch 8 oder 9, **dadurch gekennzeichnet, dass** die Abdeckung (3) bei einem Ausatemdruck durch die Phonationsöffnung von größer oder gleich 0,3 kPa gewölbt ist und der permanente Luftdurchlass (4) vergrößert ist.

11. Tracheostomiekanüle (1) nach einem der Ansprüche 1 bis 10, **dadurch gekennzeichnet, dass** das Kanülenrohr (2) einen Cuff (6) mit einem proximalen Halsabschnitt (6a) aufweist, wobei der Halsabschnitt (6a) den Cuff in Umfangsrichtung des Kanülenrohrs (2) abdichtet und die Abdeckung (3) Teil des Halsabschnittes (5a) bildet.

12. Tracheostomiekanüle (1) nach einem der Ansprüche 1 bis 11, **dadurch gekennzeichnet, dass** die Abdeckung (3) aus einem elastischen Material mit einer Shore-A-Härte von einschließlich 5 bis einschließlich 90, vorzugsweise mit einer Shore-A-Härte von einschließlich 20 bis einschließlich 60, besteht.

13. Tracheostomiekanüle (1) nach einem der Ansprüche 1 bis 12, **dadurch gekennzeichnet, dass** die Phonationsöffnung (2d) ein Phonationsfenster ist oder aus siebartig angeordneten Öffnungen besteht.

## Claims

1. A tracheostomy cannula (1) with a cannula tube (2), wherein the cannula tube (2) has a proximal end (2a) with a proximal opening, a distal end (2b) with a distal opening, as well as a phonation opening (2d) in the wall of the cannula tube (2) between the proximal end (2a) and the distal end (2b), wherein a cover (3) completely spans the opening cross section of the phonation opening (2d) and has at least one passage for air (4) which is permanently open during use of the tracheostomy cannula so that at all times air can pass from the upper natural airway through the phonation opening into the cannula tube when inhaling or out of the cannula tube through the phonation opening and the passage for air into the upper natural airway when exhaling, wherein a protrusion (5) which runs around along at least sections of the outer edge of the cover (3) functions as a spacer against the outside of the cannula tube (2) and is connected at least in parts to the cannula tube (2), wherein the protrusion (5) has the at least one permanently open passage for air (4).

2. The tracheostomy cannula (1) according to claim 1, **characterized in that** the cover (3) is set into a depression (2e) in the wall (2c) of the cannula tube (2) which surrounds the phonation opening (2d) in a manner such that the cover (3) is at least distally flush with the outer face of the cannula tube (2) or is completely disposed in the depression (2e) in the wall (2c) of the cannula tube (2).

3. The tracheostomy cannula (1) according to claim 1 or claim 2, **characterized in that** the cover (3) is disposed in the phonation opening (2d) in a manner such that the cover (3) can be brought from a first state in which the cover (3) does not protrude over the outer face of the wall (2c) of the cannula tube (2) and/or in which a part of the cover (3) protrudes into the lumen of the cannula tube (2), into a second state in which the cover (3) protrudes over the outer face of the wall (2c) of the cannula tube (2).

4. The tracheostomy cannula (1) according to claim 3, **characterized in that** an inner cannula with a phonation opening is inserted into the cannula tube (2), wherein the cover (3) and the inner cannula with phonation opening are adapted to each other in a manner such that the cover (3) can be transposed from the first state into the second state by insertion of the inner cannula with phonation opening into the cannula tube (2).

5. The tracheostomy cannula (1) according to one of claims 1 to 4, **characterized in that** the protrusion (5) consists of several protruding sections which are separated by a plurality, preferably 2 to 10, of permanently open passages for air (4) which are disposed along the circumference of the phonation opening (2d), preferably evenly distributed and diametrically opposed in pairs with respect to each other.

6. The tracheostomy cannula (1) according to one of claims 1 to 5, **characterized in that** the cover (3) has profiling on its side facing the wall (2c) of the cannula tube which extends from the outer circumference of the cover over the edge of the phonation opening (2d) and which is in engagement with the cannula tube (2).

7. The tracheostomy cannula (1) according to one of claims 1 to 6, **characterized in that** the cover (3) is bonded to, welded to and/or moulded onto the cannula tube (2).

8. The tracheostomy cannula (1) according to one of claims 1 to 7, **characterized in that** the cover (3) is secured to the cannula tube (2) along its circumference only at locations which are separated from each other, preferably on the distal and/or proximal side of the phonation opening (2d).

9. The tracheostomy cannula (1) according to one of claims 1 to 8, **characterized in that** the cover (3) has at least one permanently open passage for air (4) in the form of an aperture (3c) in the shape of a slit.

10. The tracheostomy cannula (1) according to claim 8 or claim 9, **characterized in that** when an exhalation pressure of 0.3 kPa or more is exerted through the phonation opening, the cover (3) is bulged and the permanent passage for air (4) is enlarged.

11. The tracheostomy cannula (1) according to one of claims 1 to 10, **characterized in that** the cannula tube (2) has a cuff (6) with a proximal neck section (6a), wherein the neck section (6a) seals the cuff in the circumferential direction of the cannula tube (2) and the cover (3) forms part of the neck section (5a).

12. The tracheostomy cannula (1) according to one of claims 1 to 11, **characterized in that** the cover (3) consists of an elastic material with a Shore A hardness from 5 to 90 inclusive, preferably with a Shore A hardness of 20 to 60 inclusive.

13. The tracheostomy cannula (1) according to one of claims 1 to 12, **characterized in that** the phonation opening (2d) is a phonation fenestration or consists of openings disposed in the manner of a sieve.

## Revendications

1. Canule de trachéotomie (1) avec un tube de canule (2), le tube de canule (2) comprenant une extrémité proximale (2a) avec une ouverture proximale, une extrémité distale (2b) avec une ouverture distale, ainsi qu'une ouverture de phonation (2d) dans la paroi du tube de canule (2) entre l'extrémité proximale (2a) et l'extrémité distale (2b), un couvercle (3) couvrant entièrement la section transversale de l'ouverture de phonation (2d) et comprenant au moins un passage d'air (4) qui est ouvert en permanence lors de l'utilisation de la canule de trachéotomie, de sorte que, à tout moment, de l'air provenant des voies respiratoires supérieures naturelles puisse passer, lors de l'inspiration, par l'ouverture de phonation dans le tube de canule ou, lors de l'expiration, du tube de canule par l'ouverture de phonation et le passage d'air dans les voies respiratoires supérieures naturelles, une saillie (5) qui s'étend au moins par zone le long du bord extérieur du couvercle (3), servant comme écarteur par rapport à la face extérieure du tube de canule (2) et étant attachée au moins partiellement au tube de canule (2), la saillie (5) comprenant ledit au moins un passage d'air (4) ouvert en permanence.

2. Canule de trachéotomie (1) selon la revendication 1, **caractérisée en ce que** le couvercle (3) est inséré dans un creux (2e) formé dans la paroi (2c) du tube de canule (2) et entourant l'ouverture de phonation (2d), de telle façon que, du moins du côté distal, le couvercle (3) joigne à affleurement la surface extérieure du tube de canule (2) ou soit disposé entièrement dans le creux (2e) de la paroi (2c) du tube de canule (2).

3. Canule de trachéotomie (1) selon l'une des revendications 1 ou 2, **caractérisée en ce que** le couvercle (3) est disposé dans l'ouverture de phonation (2d) de telle façon que le couvercle (3) puisse être changé d'un premier état dans lequel le couvercle (3) ne dépasse pas de la surface extérieure de la paroi (2c) du tube de canule (2) et/ou dans lequel le couvercle (3) s'étend partiellement dans la lumière du tube de canule (2), dans un deuxième état dans lequel le couvercle (3) dépasse de la surface extérieure de la paroi (2c) du tube de canule (2).

4. Canule de trachéotomie (1) selon la revendication 3, **caractérisée en ce qu'**une canule intérieure avec une ouverture de phonation est insérée dans le tube de canule (2), le couvercle (3) et la canule intérieure avec ouverture de phonation étant adaptés l'un à l'autre de telle façon que le couvercle (3) puisse être changé du premier état dans le deuxième état par l'insertion de la canule intérieure avec ouverture de phonation dans le tube de canule (2).

5. Canule de trachéotomie (1) selon l'une des revendications 1 à 4, **caractérisée en ce que** la saillie (5) est constituée par plusieurs sections de saillie qui sont séparées par une pluralité de passages d'air (4), de préférence 2 à 10, ouverts en permanence et disposés le long de la périphérie de l'ouverture de phonation (2d), de préférence répartis de façon régulière et diamétralement par paires.

6. Canule de trachéotomie (1) selon l'une des revendications 1 à 5, **caractérisée en ce que** le couvercle (3) comprend, sur son côté orienté vers la paroi (2c) du tube de canule, un profil qui s'étend du pourtour extérieur du couvercle au-delà du bord de l'ouverture de phonation (2d) et est en prise avec le tube de canule (2).

7. Canule de trachéotomie (1) selon l'une des revendications 1 à 6, **caractérisée en ce que** le couvercle (3) est collé ou soudé au, et/ou est formé d'une seule pièce avec, le tube de canule (2).

8. Canule de trachéotomie (1) selon l'une des revendications 1 à 7, **caractérisée en ce que** le couvercle (3) est fixé au tube de canule (2) le long de son pourtour uniquement à des endroits espacés les uns des autres, de préférence sur le côté distal et/ou proximal de l'ouverture de phonation (2d).

9. Canule de trachéotomie (1) selon l'une des revendications 1 à 8, **caractérisée en ce que** le couvercle (3) comprend au moins un passage d'air (4) ouvert en permanence sous la forme d'une percée (3c) en forme de fente.

10. Canule de trachéotomie (1) selon la revendication 8 ou 9, **caractérisée en ce que**, lorsque la pression d'expiration par l'ouverture de phonation est supérieure à ou égal à 0,3 kPa, le couvercle (3) est bombé et le passage d'air (4) permanent est agrandi.

11. Canule de trachéotomie (1) selon l'une des revendications 1 à 10, **caractérisée en ce que** le tube de canule (2) comprend un coussinet (6) avec une zone de cou (6a) proximale, la zone de cou (6a) rendant étanche le coussinet dans la direction périphérique du tube de canule (2) et que le couvercle (3) constitue une partie de la zone de cou (5a).

12. Canule de trachéotomie (1) selon l'une des revendications 1 à 11, **caractérisée en ce que** le couvercle (3) et fait en un matériau élastique avec une dureté Shore A allant de 5 à 90, ces deux limites incluses, de préférence avec une dureté Shore A de 20 à 60, ces deux limites incluses.

13. Canule de trachéotomie (1) selon l'une des revendications 1 à 12, **caractérisée en ce que** l'ouverture de phonation (2d) est une fenêtre de phonation ou est constituée d'ouvertures disposées à la façon d'une passoire.
